# EUROPEAN PATENT APPLICATION

(11) **EP 4 134 072 A1**
(43) Date of publication of application: **15.02.2023**
(21) Application number: 21918102.1
(22) Date of filing: 28.12.2021
(51) Int. Cl.: A61K 9/48

(54) **SOFT CAPSULE SHELL AND SOFT CAPSULE**

(30) Priority: 28.06.2021 CN 202110721182
(71) Applicant: Sirio Pharma Co., Ltd., Shantou, Guangdong 515000 (CN)
(72) Inventor: LI, Xufa, Shantou, Guangdong 515000 (CN); CHEN, Jiewei, Shantou, Guangdong 515000 (CN)
(74) Representative: Cabinet Becker et Associés
(86) International application number: PCT/CN2021/142079
(87) International publication number: WO 2023/273254

(57) **Abstract**

The invention relates to a soft capsule shell and a soft capsule. The present invention provides soft capsule shells prepared from a film-forming composition comprising 2 wt% to 5 wt% of a first gelling agent, 10 wt% to 35 wt% ofa plasticizer, 18 wt% to 35 wt% of gelatinizable starch, 4 wt% to 17 wt% of granular starch, and 35 wt% to 55 wt% of water, wherein the first gelling agent is a gellan gum having a weight-average molecular weight of 5.0 × 10⁵ to 8.0 × 10⁶ g/mol and/or a coefficient of molecular weight distribution of 1.0 to 6.0. The film-forming composition provided by the invention has good film-forming mechanical strength, toughness and viscosity, is good in seaming when being compressed into soft capsules, and can be disintegrated in medium water and simulated gastric juice. The film-forming composition is free of animal-derived components, can be applied to capsule products in the fields of medicine, food, cosmetics and the like, and is edible for all ethnic groups in the world.

## Description

### Technical Field

The invention relates to the field of food or medicine, in particular to a soft capsule shell and a soft capsule.

### Background of the Invention

Soft capsules are widely used in the fields of medicines, food products, cosmetics, etc. Gelatin is widely used as a traditional soft capsule material since it has excellent film-forming properties and mechanical strength. However, gelatin shows many drawbacks in terms of qualities during use due to its properties. For example, gelatin molecules may be self-oxidized or cross-linked with functional groups such as aldehyde groups, forming a tough and flexible water-insoluble surface film on the surface of the gelatin soft capsule, which prevents the release of drugs, resulting in the unqualified disintegration of soft capsules. In addition, due to the requirements of religions or vegetarians, and with the emergence of the mad cow disease, the foot-and-mouth disease and toxic capsules around the world, efforts are always taken to develop substitutes for gelatin and a method of their use for preparing soft capsules.

For example, the Japanese Patent No. JP2007153851A proposes a film composition for a soft capsule with non-animal origin components, which is obtained by mixing water, a starch and a natural gellan gum. The European Patent No. EP2815745A1 proposes a soft capsule comprising a high acyl gellan gum, at least one starch and at least one plasticizer, and its preparation method. The gel is formed with a soft texture and a high flexibility from high acyl gellan gum though, when in combination with starch, the ribbon is formed with a low strength, resulting in thin thickness at the seam, low rupture force, and oil leaking when shaping capsule by encapsulation. The Chinese Patent No. CN100528950C proposes a blend of different acyl gellan gums and a starch, comprising: a. a high acyl gellan gum; b. a low acyl gellan gum; c. a starch; and d. a plasticizer. The film prepared using such blends has a high modulus and excellent strength and elongation, and the soft capsules prepared have good sealability. Using compounding gellant with both high and low acyl gellan gum, the strength and toughness of ribbons can be improved to a certain degree, but the gel mass is of high viscosity and easy to pregel during delivery, resulting in defective or uneven ribbons and soft capsules prepared with thin thickness at the seam and oil leaking.

In the preparation process of soft capsules with gellan gum as the major capsule materials reported in the prior art, there are many drawbacks, such as a high viscosity of the gel mass, gelling easily in delivery, causing the ribbon defective or uneven, a thin thickness at the seam after capsule formation, and leaking oil easily, which causes the industrial production of soft capsules unachievable. There is a need in the art of vegetable soft capsules which overcome the above technical problems.

### Summary of the Invention

The present invention is based in part on the inventors' discovery that starch compositions comprising a ratio of gellan gum in particular molecular weight range to starch are better in the comprehensive effect of forming soft capsule shells, effect of disintegrating in water and gastric juices compared to starch compositions that are not within this range. Further, the inventors found that the above effect is relevant to the average particle size and the content of the starch granules in the capsule shell, that is, the content of the starch granules in the soft capsule shell that achieves the effect of the present invention ranges from 9 wt% to 40 wt%, and the starch granules are those with an average particle size of ≥10µm.

Furthermore, previous studies by the inventors have shown that the addition of pectin to the film-forming composition prevents the disintegration of the prepared soft capsule shells in the stomach. Unexpectedly, the inventors found that gellan gum in a particular molecular weight range binding starch can disintegrate in the stomach even in the presence of pectin, as shown in Table 5.

In one aspect, provided is a soft capsule shell, which is prepared from a film-forming composition comprising 2 wt% to 5 wt% of a first gelling agent, 10 wt% to 35 wt% of a plasticizer, 18 wt% to 35 wt% of gelatinizable starch, 4 wt% to 17 wt% of granular starch and 35 wt% to 55 wt% of water, wherein the first gelling agent is a gellan gum having a weight-average molecular weight of 5.0 × 10⁵ to 8.0 × 10⁶ g/mol and/or a coefficient of molecular weight distribution of 1.0 to 6.0, the content of starch granule in the soft capsule shell is 9 wt%-40 wt%, and the starch granule is a starch granule with an average particle size of ≥10 µm; the soft capsule shell is a soft capsule shell disintegrated in the stomach; the gelatinizable starch is selected from one or a combination of waxy corn starch, tapioca starch, hydroxypropyl starch, oxidized starch, oxidized hydroxypropyl starch, dextrin, or maltodextrin; the granular starch is selected from one or a combination of pea starch, corn starch, potato starch, acid-treated starch, acetate starch, hydroxypropyl distarch phosphate, starch phosphate, acetylated distarch phosphate, pregelatinized starch, acetylated distarch adipate, or acetylated oxidized starch.

In one embodiment, the gellan gum has a weight-average molecular weight of 5.5 × 10⁵ to 6.6 × 10⁶ g/mol, preferably 5.5 × 10⁵ to 4.7 × 10⁶ g/mol, and/or the gellan gum has a coefficient of molecular weight distribution of 1.0 to 4.0.

In one embodiment, the weight ratio of the gellan gum to the total amount of the starch ranges from 0.05 to 0.2, preferably the weight ratio of the gellan gum to the total amount of the starch ranges from 0.09 to 0.2; preferably, wherein the content of gellan gum ranges from 2.5 wt% to 5 wt%.

In one embodiment, the content of the plasticizer ranges from 15 wt% to 25 wt%; preferably, wherein the plasticizer is selected from one or a combination of glycerol, sorbitol, maltitol, erythritol, xylitol, crystalline fructose, trehalose or glucose.

In one embodiment, the film-forming composition further comprises 0.2 wt% to 10 wt% of a second gelling agent, preferably, the second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum.

In one embodiment, the gelatinizable starch is selected from one or both of a hydroxypropyl starch or an oxidized hydroxypropyl starch, and the granular starch is selected from one or a combination of acid-treated starch, acetate starch, hydroxypropyl distarch phosphate, starch phosphate, acetylated distarch phosphate, acetylated distarch adipate, or acetylated oxidized starch.

In one embodiment, the soft capsule shell is prepared by a method comprising a) adding a first gelling agent to a plasticizer, stirring them homogeneously, followed by adding the mixture to water, and heating them at a temperature between 60 and 98°C with stirring until the first gelling agent dissolves; b) adding the starch, and heating them at a temperature between 60 and 98°C with stirring until the starch dissolves; c) removing air bubbles to obtain a gel mass; and d) encapsulating and drying; preferably, wherein the step a) further comprises adding a second gelling agent, the step a) is a step for adding the first gelling agent and the second gelling agent to the plasticizer, stirring them homogeneously, followed by adding the mixture to water, and heating them at a temperature between 60 and 98°C with stirring until the first gelling agent and the second gelling agent dissolve.

In another aspect, the present invention provides a soft capsule comprising the soft capsule shell of the present invention and a filling.

In another aspect, the present invention provides use of the soft capsule shell or soft capsule of the present invention in food product, nutraceutical product, medicine.

In another aspect, the present invention provides use of the film-forming composition in the preparation of a soft capsule, wherein the film-forming composition comprises 2 wt% to 5 wt% of a first gelling agent, 10 wt% to 35 wt% of a plasticizer, 18 wt% to 35 wt% of gelatinizable starch , 4 wt% to 17 wt% of granular starch and 35 wt% to 55 wt% of water, wherein the first gelling agent is gellan gum having a weight-average molecular weight from 5.0 × 10⁵ to 8.0 × 10⁶ g/mol and/or a coefficient of molecular weight distribution of 1.0 to 6.0, the content of the starch granule in the soft capsule shell ranges from 9 wt% to 40 wt%, and the starch granule is a starch granule with an average particle size of ≥10 µm; the soft capsule shell is a soft capsule shell disintegrated in stomach; wherein the gelatinizable starch is selected from one or a combination of waxy corn starch, tapioca starch, hydroxypropyl starch, oxidized starch, oxidized hydroxypropyl starch, dextrin, or maltodextrin; the granular starch is selected from one or a combination of pea starch, corn starch, potato starch, acid-treated starch, acetate starch, hydroxypropyl distarch phosphate, starch phosphate, acetylated distarch phosphate, pregelatinized starch, acetylated distarch adipate or acetylated oxidized starch.

In one embodiment, the gellan gum has a weight-average molecular weight of 5.5 × 10⁵ to 6.6 × 10⁶ g/mol, preferably 5.5 × 10⁵ to 4.7 × 10⁶ g/mol, and/or the gellan gum has a coefficient of molecular weight distribution of 1.0 to 4.0.

In one embodiment, the weight ratio of the gellan gum to the total amount of the starch ranges from 0.05 to 0.2, preferably the weight ratio of the gellan gum to the total amount of the starch ranges from 0.09 to 0.2; preferably, the content of gellan gum ranges from 2.5 wt% to 5 wt%.

In one embodiment, the content of the plasticizer ranges from 15 wt% to 25 wt%; preferably, wherein the plasticizer is selected from one or a combination of glycerol, sorbitol, maltitol, erythritol, xylitol, crystalline fructose, trehalose or glucose.

In one embodiment, the film-forming composition further comprises 0.2 wt% to 10 wt% of a second gelling agent, preferably, the second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum.

In one embodiment, the gelatinizable starch is selected from one or both of hydroxypropyl starch or oxidized hydroxypropyl starch, and the granular starch is selected from one or a combination of acid-treated starch, acetate starch, hydroxypropyl distarch phosphate, starch phosphate, acetylated distarch phosphate, acetylated distarch adipate, or acetylated oxidized starch.

In one embodiment, the soft capsule shell is prepared by a method comprising a) adding a first gelling agent to a plasticizer, stirring them homogeneously, followed by adding the mixture to water, and heating them at a temperature between 60 and 98°C with stirring until the first gelling agent dissolves; b) adding the starch, and heating them at a temperature between 60 and 98°C with stirring until the starch dissolves; c) removing air bubbles to obtain a gel mass and d) encapsulating and drying.

In one embodiment, the step a) further comprises adding a second gelling agent, and the step a) is a step for adding the first gelling agent and the second gelling agent to the plasticizer, stirring them homogeneously, followed by adding the mixture to water, and heating them at a temperature between 60 and 98°C with stirring until the first gelling agent and the second gelling agent dissolve.

Herein, the film-forming composition or the soft capsule shell of the present invention does not comprise gelatin.

In one aspect, provided is use of the film-forming composition, soft capsule shell or soft capsule of the present invention in food product, nutraceutical food product, medicine and cosmetics. In one embodiment, the food product, nutraceutical food product or medicine is for gastric absorption or for gastric use. Herein, the gastric disintegration effect can be verified in simulated gastric juice.

In one aspect, the present invention provides a method of gastric delivery using the soft capsule shell or soft capsule of the present invention. The present invention also provides use of the soft capsule shell or soft capsule of the present invention in the preparation of gastric medicine.

The present invention provides advantages as follows:
1. A soft capsule shell is proposed with excellent comprehensive effect and the effect of disintegration in water and gastric juice, which enriches the formulation of soft capsule;
2. Prior to this application, it was considered that pectin should not be added to soft capsules that disintegrate in the stomach, as shown in Table 6. The present invention overcomes such cognitive bias by achieving excellent gastric disintegration in the presence of pectin through appropriate formulation and selection of gellan gum.

### Detailed Description of the Invention

The following is provided to further illustrate the present invention.

The present invention provides a soft capsule shell prepared from the film-forming composition of the present invention. The film-forming composition of the present invention comprises a first gelling agent. The content of the first gelling agent may be 2 wt%-5 wt%, such as 2 wt%, 2.5 wt%, 3 wt%, 3.5 wt%, 4 wt%, 4.5 wt%, 5 wt%. The first gelling agent may be a gellan gum. The gellan gum may have a weight-average molecular weight of 5.0 × 10⁵ to 8.0 × 10⁶ g/mol, preferably 5.5 × 10⁵ to 6.6 × 10⁶ g/mol, preferably 5.5 × 10⁵~4.7 × 10⁶ g/mol, such as 6 × 10⁵ g/mol, 9 × 10⁵ g/mol, 1.5 × 10⁶ g/mol, 2 × 10⁶ g/mol, 2.5 × 10⁶ g/mol, 3 × 10⁶ g/mol, 4 × 10⁶ g/mol, 5 × 10⁶ g/mol or 6 × 10⁶ g/mol. The gellan gum may have a coefficient of molecular weight distribution of 1.0 to 6.0, preferably a coefficient of molecular weight distribution of 1.0 to 4.0, such as 2, 2.5 or 3.

The film-forming composition comprises a plasticizer. The content of the plasticizer may be 10 wt% to 35 wt%, such as 13 wt%, 14 wt%, 15 wt%, 16 wt%, 20 wt%, 25 wt% or 30 wt%. The plasticizer may be selected from one or a combination of glycerol, sorbitol, maltitol, erythritol, xylitol, crystalline fructose, trehalose or glucose.

The film-forming composition comprises 18 to 35 wt% of gelatinizable starch and 4 to 17 wt% of granular starch. The content of the gelatinizable starch may be 20 wt% to 30 wt%, such as 20 wt%, 25 wt% or 30 wt%. The content of the granular starch may be 4 wt% to 17 wt%, such as 4 wt%, 5 wt%, 7 wt%, 8 wt%, 10 wt%, 14 wt% or 16 wt%. The gelatinizable starch refers to starch whose granules are broken or dissolved after complete gelatinization of starch; the granular starch refers to starch that does not readily gelatinize completely and whose granules are not broken after water swelling. The gelatinizable starch comprises one or a combination of waxy corn starch, tapioca starch, hydroxypropyl starch, oxidized starch, oxidized hydroxypropyl starch, dextrin or a maltodextrin. The granular starch comprises one or a combination of pea starch, corn starch, potato starch, acid-treated starch, acetate starch, hydroxypropyl distarch phosphate, starch phosphate, acetylated distarch phosphate, pregelatinized starch, acetylated distarch adipate or acetylated oxidized starch.

In one embodiment, the weight ratio of the gellan gum to the total amount of starch ranges from 0.05 to 0.2. In one embodiment, the weight ratio of the gellan gum to the total amount of starch ranges from 0.07 to 0.2. For example, the weight ratio of the gellan gum to the total amount of starch is 0.05, 0.07, 0.09, 0.1, 0.13, 0.16 or 0.2.

The film-forming composition comprises water. The content of water may be 35 wt% to 55 wt%, such as 40 wt%, 45 wt%, 50 wt% or 55 wt%.

The film-forming composition may further comprise a second gelling agent. The content of the second gelling agent may be 0.2 wt% to10 wt%, such as 0.25 wt%, 1 wt%, 2 wt%, 3 wt%, 4 wt%, 5 wt%, 6 wt%, 7 wt%, 8 wt% or 9 wt%. The second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum.

The present invention also provides a soft capsule shell prepared from the film-forming compositions of the present invention. The state of starch in the soft capsule shell is granular starch (average particle size of starch granules > 10µm), granule-broken starch (average particle size of starch granules < 10µm) or segments after starch granules break up, wherein the content of granular starch is 9 wt% to 40 wt%, such as 10 wt%, 15 wt%, 20 wt%, 25 wt%, 30 wt% or 35 wt% of the total amount of soft capsule shell. The soft capsule shell of the present invention is a soft capsule shell that disintegrates in the stomach. The inventors found that the starch granules in the soft capsule shell are related to the gastric disintegration effect of the soft capsule shell.

Further, the present invention provides a soft capsule comprising a soft capsule shell of the present invention and a filling. The filling can comprise one or a combination of various animal and vegetable fat, or suspensions, emulsions, semi-solids prepared from various solid functional ingredients and suitable excipients for soft capsules, or solid preparations (such as granule, microcapsule, powder, plain tablet, capsule) prepared from solid functional ingredients and suitable excipients.

The present invention also provides a method for preparing the soft capsule shell of the present invention, which comprises a) adding a first gelling agent to a plasticizer, stirring them homogeneously, followed by adding the mixture to water, and heating them at a temperature between 60 and 98°C with stirring until the first gelling agent dissolves; b) adding the starch, and heating them at a temperature between 60 and 98°C with stirring until the starch dissolves; c) removing air bubbles to obtain a gel mass; and d) encapsulating and drying. In one embodiment, the step a) further comprises adding a second gelling agent, the step a) is a step for adding the first gelling agent and the second gelling agent to the plasticizer, stirring them homogeneously, followed by adding the mixture to water, and heating them at a temperature between 60 and 98°C with stirring until the first gelling agent and the second gelling agent dissolve. Encapsulation can be carried out as follows: a soft capsule production line is employed and the gel mass is transported to the spreader box of the soft capsule encapsulation machine, the gel mass is cooled on the surface of a rotating drum to form a ribbon, then soft capsules are formed by extrusion and encapsulation when injecting the filling, and can be further shaped a rotating cage device. Drying can be carried out as follows: the capsules are further dried after forming or shaping, preferably dried until the moisture content of the capsule shell is 8 to 25%.

The present invention also provides use of the starch film-forming composition, soft capsule shell or soft capsule of the present invention in food product, nutraceutical product, medicine and cosmetics.

The film-forming composition of the present invention forms films with desired strength and toughness, by using the gellan gum with a particular molecular weight and distribution, in combination with starch which is controlled within a certain range. By using in the soft capsule, the film-forming composition of the present invention is obviously superior to that in the prior art in terms of the strength, toughness and adhesive property for shaping of the ribbon, which can fully meet the requirements for industrial production of soft capsules, and can meet the requirements of disintegration and rupture in medium water and artificial gastric juice, which can be used as an alternative in soft capsule technology.

The following examples are provided to further illustrate the present invention. It should be understood that the examples are only illustrative but not restrictive. The scope of the invention is defined by the appended claims.

### Examples

In order to characterize the film formed by the film-forming composition and the starch granules in the soft capsule shell, the following methods are used to measure the size and content of the granule:
1) Size of starch granule. The gel mass prepared by mixing and heating the film-forming composition is placed between two glass slides, and the gel mass is heated and pressurized to form a film at 95°C. Such film is stained by dropwise adding iodine solution after cooling. A polarizing microscope is used to randomly select multiple observation areas to take pictures. After calibrating the scale according to the magnification, the particle size of each starch granule in the photographed area is measured, and the particle size of starch is represented as arithmetic mean value.
2) Content of starch granule. 100 mg of dry film or dried soft capsule shells are dissolved with 15 g of deionized water at 75°C for 30 minutes, with stirring for several times during this period to completely dissolve the capsule shell. The turbid solution of the dissolved soft capsule shells is then centrifuged at 4000 rpm for 15min. After centrifugation under these conditions, the granular starch is in the bottom settlings, and the upper liquid is removed. After the bottom settlings are fully dried, the content of starch granule is represented as the content of settlings, and the proportion of granular starch in the soft capsule shells by mass is calculated.

In order to better illustrate the effects of the present invention, the following standards of a soft capsule are used for evaluation and description.

### (1) Capsule formation

1) Standards of the strength (F) and toughness (T) of a ribbon. A texture analyzer is used with a spherical probe and puncture mode selected, under test speed of 1.0mm/s. The rupture force is recorded when the ribbon ruptures. The greater the force, the better the strength of the ribbon. The corresponding distance (mm) upon rupture of ribbon is also recorded. The longer the distance, the better the toughness of the ribbon.
2) Standard of the adhesive property at a seam. The capsules are cut at a position other than the seam and emptied by extrusion of fillers. Then, a ring with two seams at the middle of the capsule is cut off perpendicularly to the seam, which is placed on a glass slide with the two seams perpendicular to the glass slide. The thicknesses of the two seams and the capsule shell are measured under a microscope. The ratio P (%) of the thickness of the thinnest seam to that of the capsule shell is calculated.

**Table 1 Evaluation standard of the indicators of the strength, toughness and adhesive property at a seam of the soft capsule ribbon**

| Evaluation Standards | Weight | Score | | | | | |
|---|---|---|---|---|---|---|---|
| | | 5 points | 4 points | 3 points | 2 points | 1 point | 0 point |
| strength of the ribbon (F, in N) | 20% | F ≥ 1.51N | 1.17N ≤ F< 1.51N | 0.83N ≤ F < 1.17 | 0.49N ≤ F < 0.83N | F < 0.49N | failure to form a ribbon |
| the toughness of the ribbon (T, in mm) | 20% | T ≥ 10mm | 8mm ≤ T< 10mm | 6mm ≤ T<8mm | 4mm ≤ T< 6mm | T< 4mm | failure to form a ribbon |
| the adhesive property at a seam (P, in %) | 60% | P ≥ 60% | 50% ≤ P < 60% | 40% ≤0P < 50% | 30% ≤0P < 40% | 0<P< 30% | failure to be shaped |

The feasibility of soft capsule production and formation is comprehensively evaluated using the ribbon strength (F), toughness (T) and the adhesive property at a seam (P) as standards, wherein the comprehensive evaluation is obtained by adding up 20% of score for the ribbon strength, 20% of score for toughness and 60% of score for adhesive property at a seam. The total score for the comprehensive evaluation is 5 points, and the higher the score, the better the comprehensive performance.

Both standards of ribbon strength and toughness are respectively ≥ 3 points and the standard of the adhesive property at a seam is ≥2 points, and the comprehensive evaluation must be >_2.4 to meet the industrial production of soft capsules.

### (2) Disintegration test

Referring to the regulations of the United States Pharmacopoeia USP <2040> on the disintegration test, the soft capsules are tested for disintegration in medium water and simulated gastric juice respectively, using the hanging basket device. The symbol "√" indicates the qualified disintegration in 60 minutes and the symbol "×" indicates the disqualified disintegration.

The present invention is illustrated by but not limited to the following materials, specifically as follows:
Gellan gum is a microbial metabolic gum. Differences in molecular weight and distribution of gellan gum produced by different process conditions are reflected in the differences of the rheological properties and gel properties of the gellan gum. The weight-average molecular weight (Mw) and coefficient of molecular weight distribution (Mw/Mn) of gellan gum are investigated by using SEC/MALLS (Size Exclusion Chromatography/ Multiangle Laser Light Scattering):
Gellan gum A (weight-average molecular weight (Mw): 5.98 × 10⁵ ±4.682%, coefficient of molecular weight distribution (Mw/Mn): 1.07±1.445%)
Gellan gum B (weight-average molecular weight (Mw): 9.168 × 10⁵ ±4.672%, coefficient of molecular weight distribution (Mw/Mn): 2.893 ±5.829%)
Gellan gum C (weight-average molecular weight (Mw): 1.426 × 10⁶±5.364%, coefficient of molecular weight distribution (Mw/Mn): 2.336±6.391%)
Gellan gum D (weight-average molecular weight (Mw): 2.673 × 10⁶±4.605%, coefficient of molecular weight distribution (Mw/Mn): 2.019±6.252%)
Gellan gum E (weight-average molecular weight (Mw): 4.672 × 10⁶±4.515%, coefficient of molecular weight distribution (Mw/Mn): 2.419±5.251%)
Gellan Gum F (weight-average molecular weight (Mw): 6.672 × 10⁶±4.105%, coefficient of molecular weight distribution (Mw/Mn): 2.329±5.342%)
Gellan Gum G (weight-average molecular weight (Mw): 4.01 × 10⁵±13.21%, coefficient of molecular weight distribution (Mw/Mn): 1.13±1.77%)
Gellan gum H (weight-average molecular weight (Mw): 8.608 × 10⁶±2.333%, coefficient of molecular weight distribution (Mw/Mn): 2.358±5.247%)
Gellan gum I (weight-average molecular weight (Mw): 1.503 × 10⁶±4.915%, coefficient of molecular weight distribution (Mw/Mn): 6.318±5.341%)

The method for preparing a soft capsule from the film-forming composition comprises:
1) Gel mass preparation : A) firstly premixing and dispersing a gellan gum and a plasticizer homogeneously, adding the mixture into appropriate amount of water under stirring, and then heating them at a temperature between 60 and 98°C with stirring until the gellan gum dissolves; if an edible gum other than gellan gum is included, firstly premixing and dispersing the gellan gum, the edible gum and the plasticizer homogeneously, adding the mixture into water under stirring, and then heating them at a temperature between 60 and 98°C with stirring until the gellan gum and the edible gum dissolves; B) adding a starch, and continuing to heat at a temperature between 60 and 98°C with stirring until the starch dissolves; and C) removing air bubbles to obtain a gel mass;
2) Encapsulation: Using the soft capsule production line, the gel mass is delivered to a spreader box of a soft capsule encapsulation machine and cooled on the surface of a rotating drum to form a ribbon. The soft capsules are formed by extrusion and encapsulation when injecting the filling, which can be further shaped by a rotating cage device.
3) Drying: the capsules are dried after forming or shaping, until the moisture content of the shell is 8-25%.

### Examples 1-6

Prepared with the components and contents described in Table 2, soft capsules were tested and scored. The measurement results are shown in Table 2.. According to Table 2, it can be seen that the ribbons formed by employing the combination of gellan gum with a particular molecular weight and starch in Examples 1-6 have a good strength, toughness and adhesive property upon formation, and the prepared soft capsule samples can be disintegrated with both medium water and simulated gastric juice; with the same contents of components as those of the film-forming compositions of the present invention in Control Examples 1-2, the ribbons formed by employing the combination of gellan gum with low weight-average molecular weight and starch in Control Example 1 have good strength, poor toughness and fail to be shaped by pelleting to make soft capsules; the ribbons formed by employing the combination of gellan gum with high weight-average molecular weight and starch in Control Example 2 have average strength and toughness and poor adhesive property for shaping. The ribbons formed by the combination of gellan gum with a molecular weight distribution out of the scope of the invention and starch in Control Example 3 have poor adhesive property for shaping, and can be disintegrated in both medium water and simulated gastric juice.

### Examples 7-14

Prepared with the components and contents described in Table 3, soft capsules were tested and scored. The measurement results are shown in Table 3..

In Examples 7-14, in the combination of gellan gum with a particular molecular weight and starch, as the ratio of the gellan gum increases and the ratio of starch decreases, that is, as the ratio of the gellan gum to starch gradually increases, the ribbon is formed with increased strength, slightly increased toughness, but decreased adhesive property for shaping soft capsules. It is unexpected that the comprehensive effect of starch compositions comprising gellan gum and starch in a ratio within a particular molecular weight range is superior to that of starch compositions not within this range.

### Examples 15-21

Prepared with the components and contents described in Table 4, soft capsules were tested and scored. The measurement results are shown in Table 4.Examples 15-21 illustrate that the technical effect of soft capsule prepared by the gellan gum with a particular molecular weight in combination with various types of starch is better than that of the prior art. Control Example 4 and Control Example 5 illustrate that when there is low content of starch granules in the capsule shell, the soft capsule cannot be disintegrated in the simulated gastric juice. Control Example 6 illustrates that when the starch granule is relatively high, the capsule shell is formed with poor shaping.

### Examples 22-29

Prepared with the components and contents described in Table 5 and Table 6, soft capsules were tested and scored. The measurement results are shown in Table 5..

In Examples 22-29, the technical effect of soft capsule prepared by using the gellan gum with a particular molecular weight and starch in combination with agar, locust bean gum, guar gum, konjac glucomannan, low ester pectin, carrageenan, and xanthan gum, respectively, is better than that of the prior art.

Previous studies by the inventors have shown that the addition of pectin to the film-forming composition prevents the disintegration of the prepared soft capsule shells in the stomach. Unexpectedly, the inventors found that the gellan gum within a particular molecular weight range in combination with starch can be disintegrated in the stomach even in the presence of pectin, as shown in Tables 5 and 6.

The above examples are preferred embodiments of the present invention, but the present invention is not limited to the above examples, and any other changes, modifications, substitutions, combinations, simplification made without departing from the spirit and principle of the present invention should be deemed as equivalent replacements, and are all included in the claimed scope of the present invention.

**Table 2**

| Formula composition | Example 1 | Example 2 | Example 3 | Example 4 | Example 5 | Example 6 | Control Example 1 | Control Example 2 | Control Example 3 |
|---|---|---|---|---|---|---|---|---|---|
| Gellan gum A | 3.00% | / | / | / | / | / | / | / | / |
| Gellan gum B | / | 3.00% | / | / | / | / | / | / | / |
| Gellan gum C | / | / | 3.00% | / | / | / | / | / | / |
| Gellan gum D | / | / | / | 3.00% | / | / | / | / | / |
| Gellan gum E | / | / | / | / | 3.00% | / | / | / | / |
| Gellan gum F | / | / | / | / | / | 3.00% | / | / | / |
| Gellan gum G | / | / | / | / | / | / | 3.00% | / | / |
| Gellan gum H | / | / | / | / | / | / | / | 3.00% | / |
| Gellan gum I | / | / | / | / | / | / | / | / | 3.00% |
| Hydroxypropyl starch | 21.00% | 21.00% | 21.00% | 21.00% | 21.00% | 21.00% | 21.00% | 21.00% | 21.00% |
| Hydroxypropyl distarch phosphate | 14.00% | 14.00% | 14.00% | 14.00% | 14.00% | 14.00% | 14.00% | 14.00% | 14.00% |
| Glycerol | 20.00% | 20.00% | 20.00% | 20.00% | 20.00% | 20.00% | 20.00% | 20.00% | 20.00% |
| Water | 42.00% | 42.00% | 42.00% | 42.00% | 42.00% | 42.00% | 42.00% | 42.00% | 42.00% |
| Weight ratio of the gellan gum to the starch | 0.09 | 0.09 | 0.09 | 0.09 | 0.09 | 0.09 | 0.09 | 0.09 | 0.09 |
| Strength of the ribbon | 4 | 4 | 5 | 5 | 5 | 5 | 5 | 3 | 4 |
| Toughness of the ribbon | 4 | 4 | 5 | 5 | 4 | 5 | 2 | 4 | 3 |
| Seam adhesive property upon formation | 4 | 5 | 5 | 5 | 5 | 4 | 0 | 1 | 2 |
| Comprehensive evaluation | 4 | 4.6 | 5 | 5 | 4.8 | 4.4 | 1.4 | 2 | 2.6 |
| Starch granule size (µm) | 45 | 48 | 51 | 50 | 47 | 49 | 46 | 44 | 48 |
| The percentage of starch granules in the shell by mass (%) | 14.1% | 14.0% | 14.0% | 14.1% | 14.0% | 14.0% | 14.0% | 14.1% | 14.0% |
| USP<2040> Disintegration time limit (water as the medium) | √ | √ | √ | √ | √ | √ | not applicabl e | not applicabl e | √ |
| USP<2040> Disintegration time limit (simulated gastric juice as the medium) | √ | √ | √ | √ | √ | √ | not applicabl e | not applicabl e | √ |

**Table 3**

| Formula composition | Exampl e 7 | Exampl e 8 | Exampl e 9 | Exampl e 10 | Exampl e 11 | Exampl e 12 | Exampl e 13 | Exampl e 14 |
|---|---|---|---|---|---|---|---|---|
| Gellan gum C | 2.00% | 2.50% | 3.00% | 3.50% | 4.00% | 4.00% | 4.50% | 5.00% |
| Hydroxypropyl starch | 30.00% | 20.63% | 26.25% | 22.75% | 26.25% | 22.50% | 21.00% | 18.75% |
| Hydroxypropyl distarch phosphate | 10.00% | 16.87% | 8.75% | 15.75% | 8.75% | 7.50% | 7.00% | 6.25% |
| Glycerol | 23.00% | 15.00% | 15.00% | 15.00% | 15.00% | 25.00% | 20.00% | 15.00% |
| Water | 35.00% | 45.00% | 47.00% | 43.00% | 46.00% | 41.00% | 47.50% | 55.00% |
| Weight ratio of the gellan gum to the starch | 0.05 | 0.07 | 0.09 | 0.09 | 0.11 | 0.13 | 0.16 | 0.20 |
| Strength of the ribbon | 3 | 3 | 3 | 4 | 5 | 5 | 4 | 5 |
| Toughness of the ribbon | 3 | 3 | 4 | 5 | 5 | 5 | 4 | 4 |
| Seam adhesive property upon formation | 5 | 5 | 5 | 5 | 5 | 5 | 5 | 5 |
| Comprehensive evaluation | 4.2 | 4.2 | 4.4 | 4.8 | 5 | 5 | 4.6 | 4.8 |
| Starch granule size (µm) | 55 | 60 | 48 | 52 | 57 | 60 | 70 | 65 |
| The percentage of starch granules in the shell by mass | 15.38% | 30.68% | 16.51% | 27.63% | 16.20% | 12.71% | 13.33% | 13.89% |
| USP<2040> Disintegration time limit (water as the medium) | √ | √ | √ | √ | √ | √ | √ | √ |
| USP<2040> Disintegration time limit (simulated gastric juice as the medium) | √ | √ | √ | √ | √ | √ | √ | √ |

**Table 4**

| Formula composition | Example 15 | Example 16 | Example 17 | Example 18 | Example 19 | Example 20 | Example 21 | Control Example 4 | Control Example 5 | Control Example 6 |
|---|---|---|---|---|---|---|---|---|---|---|
| Gellan gum C | 3.00% | 3.00% | 3.00% | 3.00% | 3.00% | 3.00% | 3.00% | 3.00% | 3.00% | 3.00% |
| Hydroxypropyl starch | 19.25% | / | / | 28.00% | 28.00% | 28.00% | 28.00% | 33.25% | 35.00% | 14% |
| Hydroxypropyl distarch phosphate | 15.75% | 5.25% | 10.50% | / | / | / | / | 1.75% | / | 21% |
| Acid-treated starch | / | / | / | 7.00% | / | / | / | / | / | / |
| Acetate starch | / | / | / | / | / | 7.00% | / | / | / | / |
| Oxidized hydroxypropyl starch | / | 29.75% | 24.50% | / | / | / | / | / | / | / |
| Acetylated oxidized starch | / | / | / | / | 7.00% | / | / | / | / | / |
| Acetylated distarch phosphate | / | / | / | / | / | / | 7.00% | / | / | / |
| Glycerol | 15.00% | 20.00% | 20.00% | 20.00% | 20.00% | 20.00% | 20.00% | 20.00% | 20.00% | 15.00% |
| Water | 47.00% | 42.00% | 42.00% | 42.00% | 42.00% | 42.00% | 42.00% | 42.00% | 42.00% | 47.00% |
| Weight ratio of the gellan gum to the starch | 0.09 | 0.09 | 0.09 | 0.09 | 0.09 | 0.09 | 0.09 | 0.09 | 0.09 | 0.09 |
| Strength of the ribbon | 5 | 4 | 5 | 4 | 5 | 4 | 5 | 4 | 3 | 5 |
| Toughness of the ribbon | 4 | 4 | 4 | 4 | 4 | 4 | 4 | 4 | 3 | 3 |
| Seam adhesive property upon formation | 2 | 5 | 4 | 4 | 4 | 4 | 4 | 5 | 4 | 1 |
| Comprehensive evaluation | 3 | 4.6 | 4.2 | 4 | 4.2 | 4 | 4.2 | 4.6 | 3.6 | 2.2 |
| Starch granule size (µm) | 60 | 45 | 48 | 41 | 35 | 55 | 43 | 35 | 5 | 70 |
| The percentage of starch granules in the shell by mass | 29.7% | 9.1% | 18.1% | 12.1% | 12.1% | 12.1% | 12.1% | 3.0% | 0.5% | 39.62% |
| USP<2040>Disintegration time limit (water as the medium) | √ | √ | √ | √ | √ | √ | √ | √ | √ | not applicab le |
| USP<2040>Disintegration time limit (simulated gastric juice as the medium) | √ | √ | √ | √ | √ | √ | √ | x | x | not applicab le |

**Table 5**

| Formula composition | Example 22 | Example 23 | Example 24 | Example 25 | Example 26 | Example 27 | Example 28 | Example 29 |
|---|---|---|---|---|---|---|---|---|
| Gellan gum D | 2.50% | 2.50% | 2.50% | 2.50% | 2.50% | 2.50% | 2.50% | 2.50% |
| Agar | 0.25% | / | / | / | / | / | / | / |
| Locust bean gum | / | 0.25% | / | / | / | / | / | / |
| Guar gum | / | / | 0.25% | / | / | / | / | / |
| Konjac glucomannan | / | / | / | 0.25% | | / | / | / |
| Low ester pectin | / | / | / | / | 0.25% | | | / |
| Carrageenan | / | / | / | / | / | 0.50% | | / |
| Xanthan gum | / | / | / | / | / | / | 0.25% | / |
| Hydroxypropyl distarch phosphate | 7.60% | 7.60% | 7.60% | 7.60% | 7.60% | 7.60% | 7.60% | 7.60% |
| Oxidized hydroxypropyl starch | 30.40% | 30.40% | 30.40% | 30.40% | 30.40% | 30.40% | 30.40% | 30.40% |
| Glycerol | 18% | 18% | 18% | 18% | 18% | 18% | 18% | 18% |
| Water | 41.25% | 41.25% | 41.25% | 41.25% | 41.25% | 41.00% | 41.25% | 41.50% |
| Weight ratio of the gellan gum to the starch | 0.07 | 0.07 | 0.07 | 0.07 | 0.07 | 0.07 | 0.07 | 0.07 |
| Strength of the ribbon | 5 | 5 | 4 | 4 | 4 | 5 | 4 | 4 |
| Toughness of the ribbon | 3 | 4 | 4 | 4 | 3 | 4 | 3 | 4 |
| Seam adhesive property upon formation | 3 | 3 | 3 | 3 | 3 | 3 | 3 | 4 |
| Comprehensive evaluation | 3.4 | 3.6 | 3.4 | 3.4 | 3.2 | 3.6 | 3.2 | 4 |
| Starch granule size (µm) | 55 | 53 | 48 | 50 | 47 | 51 | 54 | 49 |
| The percentage of starch granules in the shell by mass | 12.9% | 12.9% | 12.9% | 12.9% | 12.9% | 12.9% | 12.9% | 13.0% |
| USP<2040>Disintegration time limit (water as the medium) | √ | √ | √ | √ | √ | √ | √ | √ |
| USP<2040>Disintegration time limit (simulated gastric juice as the medium) | √ | √ | √ | √ | √ | √ | √ | √ |

**Table 6**

| Formula composition | | Control Example 7 | Control Example 8 | Control Example 9 | Control Example 10 | Control Example 11 | Control Example 12 |
|---|---|---|---|---|---|---|---|
| Gellan gum (total acyl content 28.0%, glyceryl 23.4%, acetyl 4.6%) (commercially available product) | | 2.50% | 3.00% | 3.50% | 2.50% | 3.00% | 3.5% |
| Low methoxyl pectin (degree of esterification DE<50%) | | / | / | / | 1.25% | 2.5% | 3.5% |
| Amide pectin (degree of amide DA 5-25%) | | 0.50% | 1.5% | 2.50% | / | / | / |
| Hydroxypropyl starch | | 17.0% | 17.0% | 17.0% | 17.0% | 17.0% | 17.0% |
| Hydroxypropyl distarch phosphate | | 17.0% | 17.0% | 17.0% | 17.0% | 17.0% | 17.0% |
| Glycerol | | 18% | 18% | 18% | 18% | 18% | 18% |
| Water | | 45.00% | 43.50% | 42.00% | 44.25% | 42.5% | 41% |
| Strength of the ribbon | | 3 | 4 | 4 | 3 | 4 | 4 |
| Toughness of the ribbon | | 3 | 3 | 3 | 3 | 3 | 4 |
| Seam adhesive property upon formation | | 3 | 3 | 3 | 3 | 3 | 2 |
| Comprehensive evaluation | | 3 | 3.2 | 3.2 | 3 | 3.2 | 2.8 |
| USP<2040>Disintegration time limit (water as the medium) | | √ | √ | √ | √ | √ | √ |
| USP<2040>Disintegration time limit (simulated gastric juice as the medium) | | x | x | x | x | x | x |
| Disintegration time limit (minutes) (According to <Chinese Pharmacopoeia> Disintegration time limit test) | First check in hydrochloric acid solution (9→1000) for 2h, the capsule shell should not have cracks or disintegration | No cracks or disintegra tion | No cracks or disintegra tion | No cracks or disintegra tion | No cracks or disintegra tion | No cracks or disintegra tion | No cracks or disintegra tion |
| | Completely disintegrated within 1h in phosphate buffer (PH6.8) | 23 minutes | 24 minutes | 20 minutes | 22 minutes | 18 minutes | 17 minutes |

## Claims

1. A soft capsule shell prepared from a film-forming composition comprising 2 wt% to 5 wt% of a first gelling agent, 10 wt% to 35 wt% of a plasticizer, 18 wt% to 35 wt% of gelatinizable starch, 4 wt% to 17 wt% of granular starch and 35 wt% to 55 wt% of water, wherein the first gelling agent is a gellan gum having a weight-average molecular weight of 5.0 × 10⁵ to 8.0 × 10⁶ g/mol and/or a coefficient of molecular weight distribution of 1.0 to 6.0, the content of starch granule in the soft capsule shell ranges from 9 wt% to 40 wt%, and the starch granule is a starch granule with an average particle size of ≥10 µm; the soft capsule shell is a soft capsule shell disintegrated in stomach;
wherein the gelatinizable starch is selected from one or a combination of hydroxypropyl starch, waxy corn starch, tapioca starch, oxidized starch, an oxidized hydroxypropyl starch, dextrin, or maltodextrin; and
wherein the granular starch is selected from one or a combination of hydroxypropyl distarch phosphate, pea starch, corn starch, potato starch, acid-treated starch, acetate starch, starch phosphate, acetylated distarch phosphate, pregelatinized starch, acetylated distarch adipate, or acetylated oxidized starch.

2. The soft capsule shell of claim 1, wherein the gellan gum has a weight-average molecular weight of 5.5 × 10⁵ to 6.6 × 10⁶ g/mol, preferably 5.5 × 10⁵ to 4.7 × 10⁶ g/mol, and/or the gellan gum has a coefficient of molecular weight distribution of 1.0 to 4.0.

3. The soft capsule shell of any one of the preceding claims, wherein the weight ratio of the gellan gum to the total amount of the starch ranges from 0.05 to 0.2, preferably the weight ratio of the gellan gum to the total amount of the starch ranges from 0.09 to 0.2; preferably, wherein the content of gellan gum ranges from 2.5 wt% to 5 wt%.

4. The soft capsule shell of any one of the preceding claims, wherein the content of the plasticizer ranges from 15 wt% to 25 wt%; preferably, wherein the plasticizer is selected from one or a combination of glycerol, sorbitol, maltitol, erythritol, xylitol, crystalline fructose, trehalose or glucose.

5. The soft capsule shell of any one of the preceding claims, wherein the film-forming composition further comprises 0.2 wt% to 10 wt% of a second gelling agent, preferably, the second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum.

6. The soft capsule shell of any one of the preceding claims, wherein the gelatinizable starch is selected from one or both of hydroxypropyl starch or oxidized hydroxypropyl starch, and the granular starch is selected from one or a combination of acid-treated starch, acetate starch, hydroxypropyl distarch phosphate, starch phosphate, acetylated distarch phosphate, acetylated distarch adipate, or acetylated oxidized starch.

7. The soft capsule shell of any one of the preceding claims, prepared by a method comprising a) adding a first gelling agent to a plasticizer, stirring them homogeneously, followed by adding the mixture to water, and heating them at a temperature between 60 and 98°C with stirring until the first gelling agent dissolves; b) adding the starch, and heating them at a temperature between 60 and 98°C with stirring until the starch dissolves; c) removing air bubbles to obtain a gel mass; and d) encapsulating and drying; preferably, wherein the step a) further comprises adding a second gelling agent, the step a) is a step for adding the first gelling agent and the second gelling agent to the plasticizer, stirring them homogeneously, followed by adding the mixture to water, and heating them at a temperature between 60 and 98°C with stirring until the first gelling agent and the second gelling agent dissolve.

8. A soft capsule comprising the soft capsule shell of any one of claims 1-7 and a filling.

9. Use of the soft capsule shell of any one of claims 1-7 or the soft capsule of claim 8 in food products, nutraceutical products, and medicines.

10. Use of a film-forming composition in the preparation of a soft capsule, wherein the film-forming composition comprises 2 wt% to 5 wt% of a first gelling agent, 10 wt% to 35 wt% of a plasticizer, 18 wt% to 35 wt% of gelatinizable starch, 4 wt% to 17 wt% of granular starch and 35 wt% to 55 wt% of water, wherein the first gelling agent is a gellan gum having a weight-average molecular weight of 5.0 × 10⁵ to 8.0 × 10⁶ g/mol and/or a coefficient of molecular weight distribution of 1.0 to 6.0, the content of starch granule in the soft capsule shell ranges from 9 wt% to 40 wt%, and the starch granule is a starch granule with an average particle size of ≥10 µm; the soft capsule shell is a soft capsule shell disintegrated in stomach; wherein the gelatinizable starch is selected from one or a combination of waxy corn starch, tapioca starch, hydroxypropyl starch, oxidized starch, oxidized hydroxypropyl starch, dextrin, or maltodextrin; the granular starch is selected from one or a combination of pea starch, corn starch, potato starch, acid-treated starch, acetate starch, hydroxypropyl distarch phosphate, starch phosphate, acetylated distarch phosphate, pregelatinized starch, acetylated distarch adipate or acetylated oxidized starch;
preferably, wherein the gellan gum has a weight-average molecular weight of 5.5 × 10⁵ to 6.6 × 10⁶ g/mol, preferably 5.5 × 10⁵ to 4.7 × 10⁶ g/mol, and/or the gellan gum has a coefficient of molecular weight distribution of 1.0 to 4.0;
preferably, wherein the weight ratio of the gellan gum to the total amount of the starch ranges from 0.05 to 0.2, preferably the weight ratio of the gellan gum to the total amount of the starch ranges from 0.09 to 0.2; preferably, wherein the content of the gellan gum ranges from 2.5 wt% to 5 wt%;
preferably, wherein the content of the plasticizer ranges from 15 wt% to 25 wt%; preferably, wherein the plasticizer is selected from one or a combination of glycerol, sorbitol, maltitol, erythritol, xylitol, crystalline fructose, trehalose or glucose;
preferably, wherein the film-forming composition further comprises 0.2 wt% to 10 wt% of a second gelling agent, preferably, the second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum;
preferably, wherein the gelatinizable starch is selected from one or both of hydroxypropyl starch or oxidized hydroxypropyl starch, and the granular starch is selected from one or a combination of acid-treated starch, acetate starch, hydroxypropyl distarch phosphate, starch phosphate, acetylated distarch phosphate, acetylated oxidized starch or acetylated distarch adipate;
preferably, wherein the soft capsule shell is prepared by a method comprising a) adding a first gelling agent to a plasticizer, stirring them homogeneously, followed by adding the mixture to water, and heating them at a temperature between 60 and 98°C with stirring until the first gelling agent dissolves; b) adding the starch, and heating them at a temperature between 60 and 98°C with stirring until the starch dissolves; c) removing air bubbles to obtain a gel mass; and d) encapsulating and drying;
preferably, wherein the step a) further comprises adding a second gelling agent, and the step a) is a step for adding the first gelling agent and the second gelling agent to the plasticizer, stirring them homogeneously, followed by adding the mixture to water, and heating them at a temperature between 60 and 98°C with stirring until the first gelling agent and the second gelling agent dissolve.
